# EUROPÄISCHE PATENTSCHRIFT

(11) **EP 4 013 322 B1**
(45) Veröffentlichungstag und Bekanntmachung des Hinweises auf die Patenterteilung: **28.08.2024**
(21) Anmeldenummer: 20756846.0
(22) Anmeldetag: 12.08.2020
(51) Int. Cl.: A61B 17/80

(54) **PLATTE ZUR TEMPORÄREN ÜBERBRÜCKUNG VON FRAGMENTEN EINER FRAKTUR**
PLATE FOR TEMPORARY BRIDGING OF FRAGMENTS OF A FRACTURE
PLAQUE DE PONTAGE TEMPORAIRE DES FRAGMENTS D'UNE FRACTURE

(30) Priorität: 16.08.2019 EP 19192018
(43) Veröffentlichungstag der Anmeldung: 22.06.2022
(73) Patentinhaber: Medartis Holding AG, 4057 Basel (CH)
(72) Erfinder: EDWARDS, Scott Graham, Scottsdale, Arizona 85259 (US); EBI, Daniel, 4435 Niederdorf (CH); FASCHIAN, Julia, 79725 Laufenburg (DE); SCHÄTZLE, Simon Martin, 79288 Gottenheim (DE)
(74) Vertreter: Hepp Wenger Ryffel AG
(86) Internationale Anmeldenummer: PCT/EP2020/072657
(87) Internationale Veröffentlichungsnummer: WO 2021/032571

(56) Entgegenhaltungen:
- EP-A1- 2 606 843
- US-A1- 2004 102 777
- US-A1- 2009 248 084
- US-A1- 2010 131 013
- US-A1- 2010 217 327

## Beschreibung

Die Erfindung betrifft eine Platte zur temporären Überbrückung von Fragmenten einer Fraktur.

Auf dem Gebiet der Behandlung von Gelenksstörungen, zum Beispiel nach chirurgischen Eingriffen zum Wiederausrichten von falsch ausgerichteten oder deformierten Handgelenksknochen, kann ein Chirurg eine Gelenksplatte implantieren. Mit der Platte werden in der Regel ein oder mehrere Knochenbefestigungselemente an gewünschten Stellen der Gelenksknochen angebracht. Die Platte wird in der gewünschten Position fixiert, so dass die Knochen des Gelenks die gewünschten Ausrichtungen in Bezug aufeinander haben. Das komplizierte Netzwerk von Sehnen, Muskeln und Nervenenden im Gelenk kann eine ordnungsgemäße Fixierung erschweren. Der Chirurg muss beim Fixieren der Gelenkplatte so gut wie möglich darauf achten, das umliegende Gewebe nicht zu beschädigen.

Aus der DE 10 2008 039 693 A1 ist eine Handgelenksarthrosenplatte bekannt, die einen verbreiterten Handwurzelknochenfixierabschnitt aufweist mit Durchgangslöchern, die quer zur Längsrichtung der Handgelenksarthrosenplatte beabstandet sind. Um Weichteilstörungen zu vermeiden, weist die Handgelenksarthrosenplatte eine Materialstärke von unter 3 mm auf. Um das Handgelenk in einer Entspannungssituation zu fixieren, ist der Handwurzelknochenfixierabschnitt ballig ausgebildet, erhebt sich also dorsal. Der Abschnitt kann auch löffelförmig ausgebildet sein.

In der EP 2 606 843 B1 ist eine Handgelenks-Fusionsplatte gezeigt, deren Mittelteil in palmarer Richtung von der Längsachse gebogen ist. Sie offenbart die Merkmale des Oberbegriffs des Anspruchs 1.

Die US 5,853,413 zeigt eine Handgelenk-Fusionsplatte mit einem proximalen und einem distalen Ende, die sich jeweils von einem Sattelbereich wegerstrecken und je eine Längsachse definieren. Die Längsachse des distalen Endes ist in medial-lateraler Richtung nicht axial zur Längsachse des proximalen Endes ausgerichtet.

Bei grossräumigen komplexen Frakturen (Polytrauma) kann entschieden werden, die Fragmente gar nicht detailliert zu versorgen, sondern nur temporär zu überbrücken, da man den Patienten nicht noch mehr belasten möchte. Durch äussere Reposition wird versucht, die einzelnen Knochenstücke in ihre ursprüngliche Lage zu bringen (Ligamentotaxis). Sie heilen dann dort mehr oder weniger gut aus. Zur Überbrückung dieser Trümmerzone kommen sogenannte Spannplatten zum Einsatz. Am Handgelenk wird beispielweise der Radiusschaft mit der Mittelhand verbunden, wobei die Finger beweglich bleiben können.

Für die temporäre Überbrückung eines Handgelenks werden zumeist einfach geformte, sich in einer Längsrichtung erstreckende Platten verwendet.

Es ist die Aufgabe der Erfindung, eine Platte zur temporären Überbrückung von Fragmenten einer Fraktur vorzustellen, welche die Nachteile des Bekannten vermeidet und welche insbesondere dafür sorgt, dass Irritationen von Weichteilen ausgeschlossen oder zumindest minimiert werden.

Die Aufgabe wird gelöst durch eine Platte zur temporären Überbrückung von Fragmenten einer Fraktur mit den Merkmalen des unabhängigen Anspruchs.

Die Platte dient bevorzugt zum Überbrücken von Knochenfragmenten eines Säugetiers, insbesondere eines Menschen.

Die Platte weist einen proximalen Abschnitt, einen distalen Abschnitt und einen Zwischenabschnitt auf, der zwischen dem proximalen Abschnitt und dem distalen Abschnitt angeordnet ist.

Der proximale Abschnitt besitzt eine proximale Befestigungszone, die dazu ausgebildet ist, an einem ersten Röhrenknochen angelegt und befestigt zu werden.

Die proximale Befestigungszone liegt insbesondere im proximalen Bereich der Platte und nimmt maximal 30 % bis 50 % und insbesondere maximal 25 % bis 30 % der Gesamtlänge der Platte ein.

Alternativ oder zusätzlich ist die proximale Befestigungszone durch denjenigen proximalen Bereich der Platte gebildet, in dem sich Durchgangslöcher befinden, deren jeweiliger Abstand zueinander die Länge von 20 mm nicht überschreitet. Mit anderen Worten heisst dies, dass im proximalen Bereich der Platte auch in anderen Abschnitten Durchgangslöcher vorhanden sein können. Die Befestigungszone wird aber nur durch eine Gruppe von Durchgangslöchern definiert, welche ausreichend nah beieinander liegen.

Die proximale Befestigungszone kann auch als derjenige Bereich des proximalen Abschnitts betrachtet werden, welcher im Wesentlichen keine Krümmung aufweist.

Der proximale Abschnitt kann in Draufsicht und/oder in seitlicher Ansicht eine erste Längsachse definieren, entlang derer er sich im Wesentlichen erstreckt.

Die Längsachse eines Abschnitts kann durch eine lineare Interpolation von Schwerpunkten von Schnittflächen der Platte definiert werden, wobei die Schnitte senkrecht zur wesentlichen Ausbreitungsrichtung des Abschnitts erfolgen. Die Schnittflächen können beispielsweise Konturen aufweisen, welche durch die Breite und die Dicke der Platte in der Schnittebene gegeben sind.

Die proximale Befestigungszone ist bevorzugt mit Durchgangslöchern ausgestattet, in welche ein Befestigungselement, wie ein Nagel, eine Schraube oder Draht, eingeführt werden kann.

Der distale Abschnitt weist eine distale Befestigungszone auf, die dazu ausgebildet ist, an einem zweiten Röhrenknochen angelegt und befestigt zu werden.

Die distale Befestigungszone liegt insbesondere im distalen Bereich der Platte und nimmt maximal 30 % bis 50 % und insbesondere maximal 25 % bis 30 % der Gesamtlänge der Platte ein.

Alternativ oder zusätzlich ist die distale Befestigungszone durch denjenigen distalen Bereich der Platte gebildet, in dem sich Durchgangslöcher befinden, deren jeweiliger Abstand zueinander die Länge von 20 mm nicht überschreitet. Mit anderen Worten heisst dies, dass im distalen Bereich der Platte auch in anderen Abschnitten Durchgangslöcher vorhanden sein können. Die Befestigungszone wird aber nur durch eine Gruppe von Durchgangslöchern definiert, welche ausreichend nah beieinander liegen.

Die distale Befestigungszone kann auch als derjenige Bereich des distalen Abschnitts betrachtet werden, welcher im Wesentlichen keine Krümmung aufweist.

Der distale Abschnitt kann in Draufsicht und/oder in seitlicher Ansicht eine zweite Längsachse definieren, entlang derer er sich im Wesentlichen erstreckt. Die Längsachse kann sich durch eine lineare Interpolation der Schnittflächenschwerpunkte ergeben.

Die distale Befestigungszone ist bevorzugt mit Durchgangslöchern ausgestattet, in welche ein Befestigungselement, wie ein Nagel, eine Schraube oder Draht, eingeführt werden kann.

Der Zwischenabschnitt ist dazu ausgebildet, ein Gelenk zwischen dem ersten Röhrenknochen und dem zweiten Röhrenknochen und dabei mindestens einen weiteren Knochen zu überbrücken.

Insbesondere erfolgt die Überbrückung ohne Befestigung an dem mindestens einen weiteren Knochen im überbrückten Bereich. In dem Zwischenabschnitt braucht also kein Durchgangsloch angeordnet sein. Er kann frei von Durchgangslöchern zur Aufnahme von Befestigungselementen sein.

Die proximale Befestigungszone kann beispielsweise dazu ausgebildet sein, an einem Radius angelegt und befestigt zu werden, während die distale Befestigungszone dazu ausgebildet sein kann, an einem Mittelhandknochen angelegt und befestigt zu werden.

Alternativ kann die proximale Befestigungszone dazu ausgebildet sein, an einer Tibia oder einem Talus, und die distale Befestigungszone an einem Mittelfussknochen befestigt zu werden. Die nachfolgend angegebenen Dimensionen beziehen sich, sofern nichts anderes angegeben ist, auf Anwendungen am Radius und an Mittelhandknochen. Für Anwendungen an Tibia bzw. Talus und Mittelfussknochen sind die Dimensionen der Platte entsprechend den Grössenverhältnissen anzupassen.

Die Platte weist erfindungsgemäss zwischen der proximalen Befestigungszone und der distalen Befestigungszone mindestens einen gebogenen Bereich auf.

Unter einem gebogenen Bereich wird ein Bereich verstanden, in dem die Platte mindestens einmal, bevorzugt mindestens zweimal ihre Richtung bezüglich einer Ausrichtungsachse ändert, zum Beispiel bezüglich der ersten oder zweiten Längsachse.

Die Platte ist vorzugsweise im Wesentlichen als länglicher, flächiger Körper ausgebildet, mit einer Länge, die grösser als die Breite ist, insbesondere mehr als 10-mal, und einer Dicke oder Materialstärke, die kleiner als die Breite ist. Die Breite liegt zum Beispiel zwischen 2 mm und 25 mm, bevorzugt zwischen 5 mm und 15 mm, und die Materialstärke liegt zum Beispiel zwischen 1 mm und 5 mm, bevorzugt zwischen 2,8 mm und 3,8 mm, besonders bevorzugt 3,4 mm.

Die Platte weist typischerweise eine Gesamtlänge von 10 cm bis 30 cm, bevorzugt 15 bis 25 cm, besonders bevorzugt von etwa 20 cm auf.

Die Platte ist bevorzugt aus Metall, zum Beispiel Titan oder einer Titanlegierung oder aus Implantatstahl gefertigt. Denkbar sind auch andere biokompatible Werkstoffe, insbesondere auch Karbon, implantierbare Kunststoffe und Kombination davon.

Die Platte erstreckt sich in Draufsicht im Wesentlichen in einer Ausrichtung, die bevorzugt mit der Längsrichtung eines der Röhrenknochen zusammenfallen kann, zum Beispiel des proximalen und/oder distalen Röhrenknochens.

In seitlicher Sicht weist die Platte bevorzugt zwei Längsachsen entsprechend den Längsachsen des proximalen und distalen Abschnitts auf, die einen Winkel gemäss der gewünschten Gelenkfixierung einschliessen.

Bei einem geradlinig zu fixierenden Gelenk können die Längsachsen zumindest in seitlicher Sicht zusammenfallen oder parallel zueinander verlaufen.

Erfindungsgemäß ist ein erster gebogener Bereich in Draufsicht gesehen gekrümmt.

Die Draufsicht ist auf die Seite des Plattenkörpers gerichtet, auf welcher die Länge und die Breite ersichtlich sind. Bevorzugt ist der erste gebogene Bereich Z-förmig oder U-förmig. Bei einer Z-förmigen Krümmung ändert sich die Ausrichtung zweimal in einer Richtung vom proximalen Ende zum distalen Ende, bei einer U-förmigen Krümmung erfolgt dreimal eine Richtungsänderung bezüglich einer wesentlichen Ausrichtung des proximalen und/oder distalen Abschnitts, insbesondere der jeweiligen Längsachse.

Der erste gebogene Bereich ist in Draufsicht gesehen gekrümmt ausgebildet, dass Irritationen von Weichteilen ausgeschlossen oder zumindest minimiert werden, dass insbesondere Platz für eine Streck- oder Beugesehne bereitgestellt wird.

Werden die Sehnen während der Ruhigstellung des Handgelenkes ständig gereizt, so kann dies zu einer Verletzung der Sehnen und Versteifung der Finger führen. Der gebogene Bereich sorgt dafür, dass die Sehnen Platz haben und nicht durch die Platte beeinträchtigt werden.

Ein in Draufsicht U-förmiger Bereich kann zum Beispiel eingesetzt werden, wenn ein Radius mit einem Mittelfingerknochen verbunden wird. Die Krümmung lässt Spielraum für die Fingersehnen. Ein in Draufsicht Z-förmig gekrümmter Bereich kann zum Beispiel eingesetzt werden, wenn ein Radius mit einem Zeigefingerknochen oder einem Ringfingerknochen verbunden wird. Die Krümmung lässt Spielraum für die Fingersehnen.

Die Krümmung des ersten gebogenen Bereichs kann eine Richtungsänderung mit einem Innenradius (Ri1) von 15 mm bis 22 mm an der Innenkante aufweisen, insbesondere von 19 mm. Insbesondere weisen alle Richtungsänderungen einen entsprechenden Innenradius auf.

Die Krümmung des ersten gebogenen Bereichs kann alternativ oder zusätzlich so gestaltet werden, dass ein lateraler/seitlicher Versatz entsteht, der mindestens der halben Breite der Platte entspricht, typischerweise mindestens etwa 3 mm beträgt.

Der erste gebogene Bereich kann geradlinig verlaufende Zwischenabschnitte aufweisen.

Der erste gebogene Bereich kann alternativ oder zusätzlich in Richtung vom proximalen Ende zum distalen Ende eine erste Richtungsänderung um einen Winkelbetrag von 10° bis 60°, eine zweite Richtungsänderung um einen Winkelbetrag von 10° bis 60° und gegebenenfalls eine dritte Richtungsänderung um einen Winkelbetrag von 10° bis 60° aufweisen. Typischerweise ändert sich die Richtung jeweils derart, sodass sich in Draufsicht eine Z-Form oder eine U-Form bildet.

Die Platte ist bevorzugt derart ausgebildet, dass der erste gebogene Bereich an dem distalen Ende des ersten Röhrenknochens positionierbar ist.

Der erste gebogene Bereich kann im proximalen Abschnitt liegen und kann auch Durchgangslöcher für die Befestigung aufweisen.

Bevorzugt ist mindestens ein Befestigungsloch in mindestens einem Bereich vorgesehen, in dem eine Richtungsänderung stattfindet, bevorzugt im Bereich der zweiten Richtungsänderung.

Der Ort der zweiten Richtungsänderung des ersten gebogenen Bereichs, wo typischerweise eine grösste laterale Auslenkung der Platte vorliegt, weist einen Abstand zwischen 110 mm bis 130 mm, bevorzugt von 120 mm vom proximalen Ende der Platte auf.

Alternativ oder zusätzlich kann die Platte so ausgebildet sein, dass der Ort der zweiten Richtungsänderung des ersten gebogenen Bereichs sich in einen Abstand zwischen 10 mm bis 12 mm, bevorzugt von 11 mm vom distalen Ende des ersten Röhrenknochens (1) anordnen lässt.

Dies bedeutet, dass der Ort der zweiten Richtungsänderung einen Abstand von 10 mm bis 12 mm, bevorzugt von etwa 11 mm vom proximalen Ende des Zwischenabschnitts aufweist.

Alternativ oder zusätzlich kann die zweite Richtungsänderung in einem Abstand von 25 mm bis 45 mm, bevorzugt 30 mm bis 40 mm von der ersten Richtungsänderung angeordnet sein.

In einer vorteilhaften Ausführung der Platte weist die Platte einen zweiten gebogenen Bereich auf, der in Seitenansicht gekrümmt ist.

Die Seitenansicht ist auf die Seite des Plattenkörpers gerichtet, auf welcher die Länge und die Dicke ersichtlich sind. Bevorzugt ist der zweite gebogene Bereich Z-förmig oder U-förmig gekrümmt. Die Krümmung erstreckt sich bevorzugt von der gegen den Knochen richtbaren Unterseite der Platte. Bei einer U-förmigen Krümmung erstrecken sich die Schenkel und die Basis des U-förmigen Querschnitts daher insbesondere von der gegen den Knochen hin richtbaren Unterseite der Platte, so dass auf der Oberseite aufgrund der zweiten Krümmung einer Vertiefung entsteht.

Der zweite gebogene Bereich ist bevorzugt im Zwischenabschnitt angeordnet. Der zweite gebogene Bereich enthält bevorzugt keine Durchgangslöcher und ist somit nicht dafür vorgesehen, an einem Knochen fixiert zu werden.

Eine Platte mit erstem und zweitem gebogenen Bereich kann also um die Weichteile herumgeführt werden und diesen Platz für die Bewegung lassen.

Der zweite gebogene Bereich erlaubt es, dass die Platte bei der Anbringung automatisch die optimale Lage einnimmt, sofern eine für den Patienten passende Grösse der Platte gewählt wurde.

Ist die Platte als Handgelenksplatte ausgeführt, so ist sie am distalen Radius in den Strecksehnenfächern positionierbar.

Die Krümmung des zweiten gebogenen Bereichs kann eine Richtungsänderung mit einem Innenradius (Ri2) von 12 mm bis 18 mm an der zur Innenkrümmung weisenden Kante der Platte aufweisen, insbesondere von 12 mm. Insbesondere weisen alle Richtungsänderungen einen solchen Innenradius auf.

Die Krümmung des zweiten gebogenen Bereichs kann alternativ oder zusätzlich so gewählt werden, dass der vertikale Versatz (d.h. der Versatz bezüglich der Plattenebene) 1 mm bis 10 mm, bevorzugt 3 mm bis 4 mm beträgt. Der zweite gebogene Bereich kann geradlinig verlaufende Zwischenabschnitte aufweisen.

Der zweite gebogene Bereich kann in Richtung vom proximalen Ende zum distalen Ende eine erste Richtungsänderung um einen Winkelbetrag von 10° bis 60°, eine zweite Richtungsänderung um einen Winkelbetrag von 10° bis 60° und gegebenenfalls eine dritte Richtungsänderung um einen Winkelbetrag von 10° bis 60° aufweisen. Typischerweise ändert sich die Richtung jeweils derart, sodass sich in Seitenansicht eine Z-Form oder eine U-Form bildet.

Die Richtungsänderungen erfolgen bevorzugt derart, dass nach einer ersten, zweiten und gegebenenfalls dritten Richtungsänderung (in einer Richtung vom proximalen Ende zum distalen Ende) die Längsachsen des proximalen und des distalen Abschnitts in Seitenansicht den Winkel der gewünschten Gelenkstellung einschliessen, also den Winkel, den die zu verbindenden Röhrenknochen einschliessen sollen.

In einer vorteilhaften Ausführung der Platte weist der zweite gebogene Bereich einen Ort der zweiten Richtungsänderung auf, wo typischerweise die grösste vertikale Auslenkung der Platte vorliegt. Dieser Ort weist zum Beispiel einen Abstand zwischen 50 mm bis 70 mm, bevorzugt von 61 mm, vom distalen Ende der Platte auf.

Alternativ oder zusätzlich ist die Platte so ausgebildet, dass sich der Ort der zweiten Richtungsänderung des zweiten gebogene Bereichs in einem Abstand zwischen 10 mm bis 15 mm, bevorzugt von 11 mm, vom proximalen Ende des zweiten Röhrenknochens anordnen lässt.

Dies bedeutet, dass der den grössten seitlichen/lateraler Abstand von dem Bereich der Platte vor der ersten Richtungsänderung aufweisende Punkt des zweiten gebogenen Bereichs einen Abstand zwischen 10 mm bis 12 mm, bevorzugt von 11 mm vom proximalen Ende des distalen Abschnitts aufweist.

In einer vorteilhaften Ausbildung der Platte sind die Durchgangslöcher in der proximalen Befestigungszone und/oder im ersten gebogenen Bereich und/oder in der distalen Befestigungszone als mindestens ein Schraubenloch, Langloch und/oder K-Drahtloch ausgebildet.

Mittels des K-Drahtlochs kann die Platte vorfixiert werden, sodass unter Röntgenkontrolle die Position vor dem Verschrauben überprüft werden kann.

Die Längsachse des proximalen Abschnitts kann mit der Längsachse des distalen Abschnitts in Draufsicht einen Winkel von +45° bis -45°, bevorzugt 0°, einschliessen.

Auch wenn die zu verbindenden Röhrenknochen in Draufsicht etwa fluchten, zum Beispiel ein Radius und ein Mittelfinger, kann es vorteilhaft sein, wenn die Längsachsen in Draufsicht einen Winkel einschliessen. Eine Längsachse kann beispielsweise entlang der Längsachse des distalen Röhrenknochens angeordnet werden, welcher in der Regel einen kleineren Durchmesser hat. Die Längsachse des proximalen Abschnitts kann in Draufsicht im befestigten Zustand gegenüber der Längsachse des proximalen Röhrenknochens je nach Knochenmodell auch geneigt sein. Eine Anzahl von Befestigungslöchern entlang der Längsachse des proximalen Endes kann auf einem kürzeren Stück des Knochens angeordnet werden. Die Platte kann also insgesamt kürzer ausgeführt werden, wobei dieselbe Befestigungsstabilität erreicht wird.

Die Längsachse des proximalen Abschnitts kann mit der Längsachse des distalen Abschnitts in seitlicher Ansicht einen Winkel von -45° bis +45° einschliessen. Für eine Handgelenksplatte schliessen die Längsachsen in seitlicher Ansicht bevorzugt einen Winkel von 12° ein.

Vorteilhafterweise nimmt die Dicke der Platte im proximalen Abschnitt zum proximalen Ende hin ab und/oder die Dicke der Platte im distalen Abschnitt nimmt zum distalen Ende hin ab. Insbesondere wird die Materialstärke einer flächigen Platte zu den Enden hin kleiner.

Die Platte lässt sich dann leichter in den Gelenksbereich einbringen und am Übergang zwischen Platte und Knochen werden Unstetigkeiten in den Biegemomenten verhindert.

Es wird ausserdem ein Verfahren zum Behandeln einer komplexen Gelenkfraktur beschrieben, wobei sich das Gelenk zwischen zwei Röhrenknochen eines menschlichen Patienten befindet. Dieses Verfahren bildet keinen Teil der beanspruchten Erfindung. Es dient lediglich dem Verständnis der Erfindung.

Zur temporären Überbrückung von Fragmenten der Fraktur wird eine Platte, bevorzugt wie oben beschrieben, bereitgestellt, mit einem proximalen Abschnitt, einem distalen Abschnitt und einem Zwischenabschnitt, der zwischen dem proximalen Abschnitt und dem distalen Abschnitt angeordnet ist.

Der proximale Abschnitt weist eine proximale Befestigungszone auf, die an einem ersten Röhrenknochen angelegt und befestigt wird.

Der distale Abschnitt weist eine distale Befestigungszone auf, die an dem zweiten Röhrenknochen angelegt und befestigt wird.

Der Zwischenabschnitt überbrückt das Gelenk zwischen dem ersten Röhrenknochen und dem zweiten Röhrenknochen und dabei mindestens einen weiteren Knochen, insbesondere ohne Befestigung an dem mindestens einen weiteren Knochen.

Die Platte zwischen der proximalen Befestigungszone und der distalen Befestigungszone weist mindestens einen gebogenen Bereich auf.

Die proximale Befestigungszone kann an einem Radius und die distale Befestigungszone an einem Mittelhandknochen angelegt und befestigt werden.

Alternativ kann die proximale Befestigungszone an einer Tibia und die distale Befestigungszone an einem Mittelfussknochen befestigt werden.

Bevorzugte Ausführungsbeispiele der Erfindung sind in der nachfolgenden Beschreibung anhand der beigefügten Zeichnungen näher erläutert.

Es zeigen
- Figur 1: eine Platte in Draufsicht;
- Figur 2: eine Platte in seitlicher Ansicht.

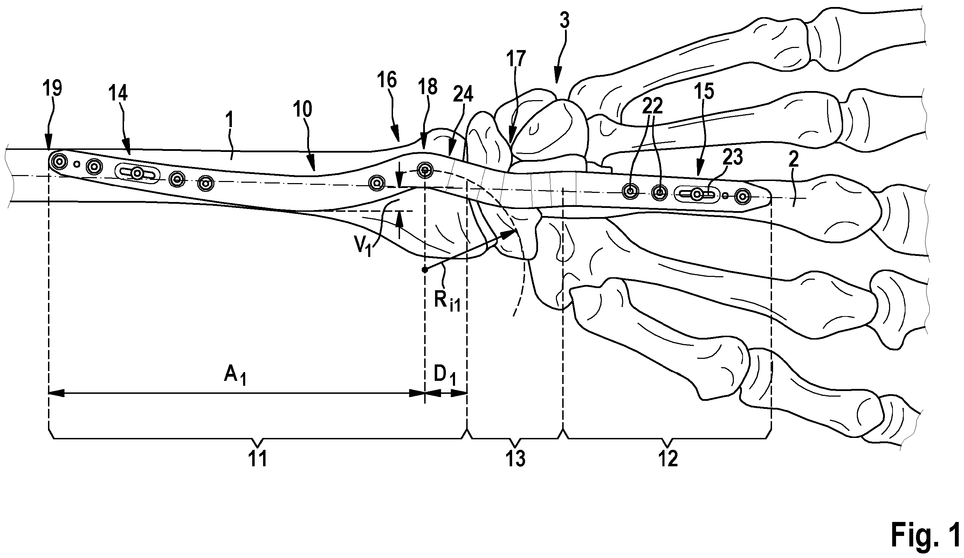

Figur 1 zeigt eine Platte 10 zur temporären Überbrückung von Fragmenten einer Handgelenksfraktur in Draufsicht. Die Platte 10 besitzt einen proximalen Abschnitt 11, einen distalen Abschnitt 12 und einen Zwischenabschnitt 13, der zwischen dem proximalen Abschnitt 11 und dem distalen Abschnitt 12 angeordnet ist.

Der proximale Abschnitt 11 weist eine proximale Befestigungszone 14 auf, die dazu ausgebildet ist, an einem ersten Röhrenknochen 1, hier ein Radius, angelegt und befestigt zu werden.

Der distale Abschnitt 12 besitzt eine distale Befestigungszone 15, die an einem zweiten Röhrenknochen 2, hier einem Mittelfingerknochen, angelegt und befestigt ist.

Der Zwischenabschnitt 13 überbrückt das Handgelenk 3 zwischen dem Radius 1 und dem Mittelfingerknochen 2 und liegt über den Handwurzelknochen.

Der Zwischenabschnitt 13 ist nicht an den Handwurzelknochen befestigt.

Die Platte 10 weist zwischen der proximalen Befestigungszone 14 und der distalen Befestigungszone 15 einen ersten gebogenen Bereich 16 auf, der in Drausicht -förmig gekrümmt ist. Der erste gebogene Bereich 16 ist an dem distalen Ende des Radius 1 positioniert.

Die Platte 10 ist daher derart ausgebildet, dass ein Einklemmen von Streck- oder Beugesehnen (hier nicht dargestellt) verhindert werden kann.

Die Krümmung des ersten gebogenen Bereichs 16 weist einen Ort 18 der zweiten Richtungsänderung mit einem Innenradius Rᵢ₁ von 15 mm bis 22 mm auf, insbesondere von 19 mm.

Der laterale Versatz V₁ entspricht etwa der mittleren Breite der Platte und beträgt etwa 10 mm.

Der Ort 18 der zweiten Richtungsänderung mit der grössten lateralen Auslenkung mit dem Versatz V₁ kann einen Abstand A₁ zwischen 110 mm bis 130 mm, bevorzugt von 120 mm vom proximalen Ende 19 der Platte 10 aufweisen.

Der Ort 18 mit der grössten lateralen Auslenkung ist bevorzugt in einem Abstand D₁ zwischen 10 mm bis 12 mm, bevorzugt von 11 mm vom distalen Ende des Radius 1 angeordnet.

In der proximalen Befestigungszone 14, im ersten gebogenen Bereich 16 und in der distalen Befestigungszone 15 sind Schraubenlöcher 22 vorgesehen. In der proximalen Befestigungszone 14 und in der distalen Befestigungszone 15 ist ausserdem jeweils ein Langloch 23 angebracht. Zusätzlich befindet sich in dem ersten gebogenen Bereich 16 ein K-Drahtloch 24.

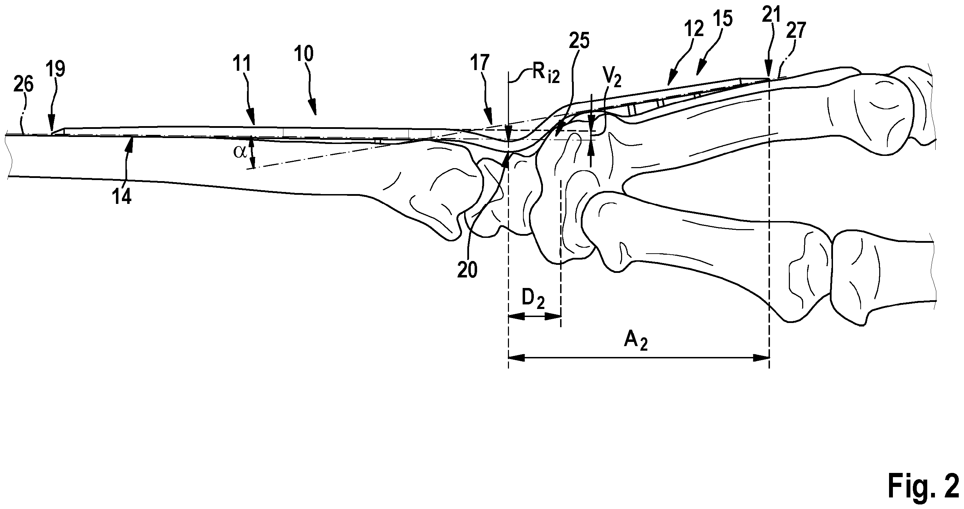

Figur 2 zeigt die Platte 10 in seitlicher Ansicht.

Die Platte 10 weist zwischen der proximalen Befestigungszone 14 und der distalen Befestigungszone 15 einen zweiten gebogenen Bereich 17 auf, der in Seitenansicht U-förmig gekrümmt ist.

Der zweite gebogene Bereich 17 kann unter die nicht in der Figur gezeigten Weichteile des Gelenks geschoben werden.

Der Ort 20 der zweiten Richtungsänderung des zweiten gebogenen Bereichs 17, wo typischerweise die grösste vertikale Auslenkung mit einem Versatz V₂ vorliegt, kann einen Innenradius Rᵢ₂ von 12 mm bis 18 mm besitzen, insbesondere von 12 mm. Der vertikale Versatz V₂ beträgt etwa 3 mm.

Der Ort 20 mit der grössten vertikalen Auslenkung kann einen Abstand A₂ zwischen 50 mm bis 70 mm, bevorzugt von 61 mm, vom distalen Ende 21 der Platte 10 aufweisen.

Der Ort 20 der zweiten Richtungsänderung des zweiten gebogenen Bereichs 17 kann in einem Abstand D₂ zwischen 10 mm bis 12 mm, bevorzugt von 11 mm vom proximalen Ende 25 des Mittefingerknochens angeordnet sein.

Die Längsachse 26 des proximalen Abschnitts 11 schliesst mit einer Längsachse 27 des distalen Abschnitts 12 in seitlicher Ansicht einen Winkel α von etwa 12° ein.

Die Dicke der Platte 10 nimmt zu den Enden 19, 21 der Platte hin ab.

| | Hand gross | Hand mittel | Hand klein |
|---|---|---|---|
| Länge (mm) | 230 | 200 | 170 |
| Breite (mm) | 12 | 10 | 8 |
| Dicke (mm) | 5 | 3,5 | 2 |
| D₁ (mm) | 13 | 11 | 9 |
| A₁ (mm) | 105 | 120 | 135 |
| D₂ (mm) | 12 | 11 | 10 |
| A₂ (mm) | 60 | 55 | 45 |
| Rᵢ₁ (mm) | 25 | 19 | 5 |
| Rᵢ₂ (mm) | 20 | 12 | 2 |
| α (°) | 12 | 12 | 12 |
| V₁ (mm) | 8 | 5 | 3 |
| V₂ (mm) | 7 | 5 | 3 |

## Patentansprüche

1. Platte (10) zur temporären Überbrückung von Fragmenten einer Fraktur mit einem proximalen Abschnitt (11), einem distalen Abschnitt (12) und einem Zwischenabschnitt (13), der zwischen dem proximalen Abschnitt (11) und dem distalen Abschnitt (12) angeordnet ist, wobei
- der proximale Abschnitt (11) eine proximale Befestigungszone (14) aufweist, die dazu ausgebildet ist, an einem ersten Röhrenknochen (1) angelegt und befestigt zu werden,
- der distale Abschnitt (12) eine distale Befestigungszone (15) aufweist, die dazu ausgebildet ist, an einem zweiten Röhrenknochen (2) angelegt und befestigt zu werden und
- der Zwischenabschnitt (13) dazu ausgebildet ist, ein Gelenk (3) zwischen dem ersten Röhrenknochen (1) und dem zweiten Röhrenknochen (2) und dabei mindestens einen weiteren Knochen, insbesondere ohne Befestigung an dem mindestens einen weiteren Knochen, zu überbrücken,
wobei
die Platte (10) zwischen der proximalen Befestigungszone (14) und der distalen Befestigungszone (15) mindestens einen gebogenen Bereich (16, 17) aufweist, **dadurch gekennzeichnet, dass** ein erster gebogener Bereich (16) in Draufsicht gesehen gekrümmt ist, bevorzugt U-förmig oder Z-förmig.

2. Platte (10) gemäss Anspruch 1, wobei die proximale Befestigungszone (14) dazu ausgebildet ist, an einem Radius angelegt und befestigt zu werden und die distale Befestigungszone (15) an einem Mittelhandknochen.

3. Platte (10) gemäss Anspruch 1, wobei die proximale Befestigungszone (14) dazu ausgebildet ist, an einer Tibia und die distale Befestigungszone (15) an einem Mittelfussknochen befestigt zu werden.

4. Platte (10) gemäss einem der vorangehenden Ansprüche, wobei die Krümmung des ersten gebogenen Bereichs (16) derart ausgebildet ist, dass Platz für eine Streck- oder Beugesehne bereitgestellt wird.

5. Platte (10) gemäss einem der vorangehenden Ansprüche,
wobei die Krümmung des ersten gebogenen Bereichs (16) mindestens eine Richtungsänderung mit einen Innenradius (Rᵢ₁) von 15 mm bis 22 mm aufweist, insbesondere von 19 mm, und/oder wobei die Krümmung des ersten gebogenen Bereichs (16) mindestens eine erste Richtungsänderung um einen Winkelbetrag von 10° bis 60° aufweist
und/oder wobei die Krümmung des ersten gebogenen Bereichs (16) zu einem lateralen Versatz V₁ führt, der mindestens 3 mm beträgt.

6. Platte (10) gemäss einem der vorhergehenden Ansprüche, wobei der erste gebogene Bereich (16) an dem distalen Ende des ersten Röhrenknochens (1) positionierbar ist.

7. Platte (10) gemäss einem der vorhergehenden Ansprüche, wobei der Ort der zweiten Richtungsänderung (18) des ersten gebogenen Bereichs (16) einen Abstand (A₁) zwischen 110 mm bis 130 mm, bevorzugt von 120 mm vom proximalen Ende (19) der Platte (10) aufweist.

8. Platte (10) gemäss einem der vorhergehenden Ansprüche, wobei die Platte (10) so ausgebildet ist, dass sich der Ort der zweiten Richtungsänderung (18) des ersten gebogene Bereichs (16) in einem Abstand (D₁) zwischen 10 mm bis 12 mm, bevorzugt von 11 mm vom distalen Ende des ersten Röhrenknochens (1) anordnen lässt.

9. Platte (10) gemäss einem der vorhergehenden Ansprüche, wobei ein zweiter gebogener Bereich (17) in Seitenansicht gekrümmt ist, bevorzugt Z-förmig oder U-förmig.

10. Platte (10) gemäss Anspruch 8 oder 9, wobei die Krümmung des zweiten gebogenen Bereichs (17) mindestens eine Richtungsänderung mit einem Innenradius (Rᵢ₂) von 12 mm bis 18 mm aufweist, insbesondere von 12 mm,
und/oder wobei die Krümmung des zweiten gebogenen Bereichs (16) mindestens eine erste Richtungsänderung um einen Winkelbetrag von 10° bis 60° aufweist,
und/oder wobei die Krümmung des zweiten gebogenen Bereichs (17) zu einem vertikalen Versatz V₂ führt, der mindestens 3 mm beträgt.

11. Platte (10) gemäss einem der Ansprüche 8 bis 10, wobei der zweite gebogene Bereich (17) im Zwischenabschnitt angeordnet ist.

12. Platte (10) gemäss einem der Ansprüche 8 bis 11, wobei der zweite gebogene Bereich (17) keine Durchgangslöcher enthält.

13. Platte (10) gemäss einem der Ansprüche 8 bis 12, wobei der Ort (20) der zweiten Richtungsänderung des zweiten gebogenen Bereichs (17) einen Abstand (A₂) zwischen 50 mm bis 70 mm, bevorzugt von 61 mm, vom distalen Ende (21) der Platte (10) aufweist
und/oder
die Platte (10) so ausgebildet ist, dass sich der Ort (20) der zweiten Richtungsänderung des zweiten gebogene Bereichs (17) in einem Abstand (D2) zwischen 10 mm bis 12 mm, bevorzugt von 11 mm vom proximalen Ende (25) des zweiten Röhrenknochens (2) anordnen lässt.

14. Platte (10) gemäss einem der vorhergehenden Ansprüche, wobei in der proximalen Befestigungszone (14) und/oder im ersten gebogenen Bereich (16) und/oder in der distale Befestigungszone (15) mindestens ein Schraubenloch (22) und/oder mindestens ein Langloch (23) und/oder mindestens ein K-Drahtloch (24) vorgesehen ist.

15. Platte (10) gemäss einem der vorhergehenden Ansprüche, wobei eine Längsachse des proximalen Abschnitts (11) mit einer Längsachse des distalen Abschnitts (12) in Draufsicht einen Winkel von +45° bis -45°, bevorzugt 0°, einschliesst und/oder in seitlicher Ansicht einen Winkel (α) von -45° bis +45°, bevorzugt 12°, einschliesst.

16. Platte (10) gemäss einem der vorhergehenden Ansprüche, wobei die Dicke der Platte (10) im proximalen Abschnitt (11) zum proximalen Ende (19) hin kleiner wird und/oder die Dicke der Platte (10) im distalen Abschnitt (12) zum distalen Ende (21) hin kleiner wird.

## Claims

1. A plate (10) for temporarily bridging fragments of a fracture comprising a proximal portion (11), a distal portion (12) and an intermediate portion (13) disposed between the proximal portion (11) and the distal portion (12), wherein
- the proximal portion (11) has a proximal attachment zone (14) adapted to be applied and attached to a first tubular bone (1),
- the distal portion (12) has a distal attachment zone (15) adapted to be applied and attached to a second tubular bone (2), and
- the intermediate portion (13) is designed to bridge a joint (3) between the first tubular bone (1) and the second tubular bone (2) and thereby at least one further bone, in particular without attachment to the at least one further bone,
wherein
the plate (10) has at least one curved region (16, 17) between the proximal fastening zone (14) and the distal fastening zone (15), **characterized in that** a first curved region (16) is curved, preferably U-shaped or Z-shaped, when viewed from above.

2. Plate (10) according to claim 1, wherein the proximal attachment zone (14) is designed to be applied and attached to a radius and the distal attachment zone (15) is designed to be applied and attached to a metacarpal bone.

3. Plate (10) according to claim 1, wherein the proximal attachment zone (14) is adapted to be attached to a tibia and the distal attachment zone (15) is adapted to be attached to a metatarsal bone.

4. Plate (10) according to one of the preceding claims, wherein the curvature of the first curved region (16) is formed such that space is provided for an extensor or flexor tendon.

5. Plate (10) according to one of the preceding claims,
wherein the curvature of the first curved region (16) has at least one change in direction with an inner radius (Rᵢ₁) of 15 mm to 22 mm, in particular of 19 mm, and/or wherein the curvature of the first curved region (16) has at least a first change in direction by an angular amount of 10° to 60° and/or wherein the curvature of the first curved region (16) leads to a lateral offset V1 which is at least 3 mm.

6. Plate (10) according to any one of the preceding claims, wherein the first curved region (16) is positionable at the distal end of the first tubular bone (1).

7. Plate (10) according to any one of the preceding claims, wherein the location of the second change of direction (18) of the first curved region (16) has a distance (A1) of between 110 mm to 130 mm, preferably of 120 mm from the proximal end (19) of the plate (10).

8. Plate (10) according to one of the preceding claims,
wherein the plate (10) is formed such that the location of the second change of direction (18) of the first curved region (16) can be arranged at a distance (D1) of between 10 mm to 12 mm, preferably of 11 mm, from the distal end of the first tubular bone (1).

9. Plate (10) according to one of the preceding claims,
wherein a second curved region (17) is curved in side view, preferably Z-shaped or U-shaped.

10. Plate (10) according to claim 8 or 9, wherein the curvature of the second curved region (17) has at least one change of direction with an inner radius (Rᵢ₂) of 12 mm to 18 mm, in particular of 12 mm, and/or wherein the curvature of the second curved region (16) has at least a first change of direction by an angular amount of 10° to 60°, and/or wherein the curvature of the second curved region (17) leads to a vertical offset V2 which is at least 3 mm.

11. Plate (10) according to any one of claims 8 to 10, wherein the second curved region (17) is arranged in the intermediate portion.

12. Plate (10) according to any one of claims 8 to 11, wherein the second curved region (17) does not include through holes.

13. Plate (10) according to any one of claims 8 to 12, wherein the location (20) of the second change of direction of the second curved region (17) is at a distance (A2) of between 50 mm to 70 mm, preferably of 61 mm, from the distal end (21) of the plate (10) and/or the plate (10) is formed such that that the location (20) of the second change in direction of the second curved region (17) can be arranged at a distance (D2) of between 10 mm and 12 mm, preferably 11 mm, from the proximal end (25) of the second tubular bone (2).

14. Plate (10) according to one of the preceding claims,
wherein at least one screw hole (22) and/or at least one slotted hole (23) and/or at least one K-wire hole (24) is provided in the proximal attachment zone (14) and/or in the first curved region (16) and/or in the distal attachment zone (15).

15. Plate (10) according to one of the preceding claims,
wherein a longitudinal axis of the proximal portion (11) encloses an angle of +45° to -45°, preferably 0°, with a longitudinal axis of the distal portion (12) in plan view and/or encloses an angle (α) of -45° to +45°, preferably 12°, in lateral view.

16. Plate (10) according to one of the preceding claims,
wherein the thickness of the plate (10) in the proximal portion (11) becomes smaller towards the proximal end (19) and/or the thickness of the plate (10) in the distal portion (12) becomes smaller towards the distal end (21).

## Revendications

1. Plaque (10) pour le pontage temporaire de fragments d'une fracture, comprenant une partie proximale (11), une partie distale (12) et une partie intermédiaire (13) disposée entre la partie proximale (11) et la partie distale (12), dans laquelle
- la partie proximale (11) comprend une zone de fixation proximale (14) adaptée pour être appliquée et fixée à un premier os tubulaire (1),
- la partie distale (12) comprend une zone de fixation distale (15) adaptée pour être placée et fixée sur un deuxième os tubulaire (2), et
- la partie intermédiaire (13) est conçue pour ponter une articulation (3) entre le premier os tubulaire (1) et le deuxième os tubulaire (2) et ainsi au moins un autre os, en particulier sans fixation sur ledit au moins un autre os,
où
la plaque (10) présente entre la zone de fixation proximale (14) et la zone de fixation distale (15) au moins une zone courbée (16, 17), **caractérisée en ce qu'**une première zone courbée (16) est courbée, de préférence en forme de U ou de Z, vue de dessus.

2. Plaque (10) selon la revendication 1, dans laquelle la zone de fixation proximale (14) est adaptée pour être appliquée et fixée à un radius et la zone de fixation distale (15) est adaptée pour être appliquée à un métacarpe.

3. Plaque (10) selon la revendication 1, dans laquelle la zone de fixation proximale (14) est adaptée pour être fixée à un tibia et la zone de fixation distale (15) est adaptée pour être fixée à un métatarsien.

4. Plaque (10) selon l'une quelconque des revendications précédentes, dans laquelle la courbure de la première zone courbée (16) est telle qu'elle fournit un espace pour un tendon d'étirement ou de flexion.

5. Plaque (10) selon l'une quelconque des revendications précédentes, dans laquelle la courbure de la première zone courbée (16) présente au moins un changement de direction avec un rayon interne (Rᵢ₁) de 15 mm à 22 mm, en particulier de 19 mm,et/ou la courbure de la première zone courbée (16) présentant au moins un premier changement de direction d'une valeur angulaire de 10° à 60° et/ou dans lequel la courbure de la première zone courbe (16) entraîne un décalage latéral V1 qui est d'au moins 3 mm.

6. Plaque (10) selon l'une quelconque des revendications précédentes, dans laquelle la première zone courbée (16) peut être positionnée à l'extrémité distale du premier os tubulaire (1).

7. Plaque (10) selon l'une quelconque des revendications précédentes, dans laquelle l'emplacement du second changement de direction (18) de la première partie courbée (16) est à une distance (A1) comprise entre 110 mm et 130 mm, de préférence de 120 mm, de l'extrémité proxiale (19) de la plaque (10).

8. Plaque (10) selon l'une quelconque des revendications précédentes, dans laquelle la plaque (10) est configurée pour permettre de placer l'emplacement du deuxième changement de direction (18) de la première zone courbée (16) à une distance (D1) comprise entre 10 mm et 12 mm, de préférence de 11 mm, de l'extrémité distale du premier os tubulaire (1).

9. Plaque (10) selon l'une quelconque des revendications précédentes, dans laquelle une seconde zone courbée (17) est courbée en vue latérale, de préférence en forme de Z ou de U.

10. Plaque (10) selon la revendication 8 ou 9, dans laquelle la courbure de la deuxième zone courbée (17) présente au moins un changement de direction avec un rayon intérieur (Rᵢ₂) de 12 mm à 18 mm, en particulier de 12 mm, et/ou dans laquelle la courbure de la deuxième zone courbée (16) présente au moins un premier changement de direction d'une valeur angulaire de 10° à 60°, et/ou dans laquelle la courbure de la deuxième zone courbée (17) conduit à un décalage vertical V2 qui est d'au moins 3 mm.

11. Plaque (10) selon l'une quelconque des revendications 8 à 10, dans laquelle la deuxième zone courbée (17) est située dans la partie intermédiaire.

12. Plaque (10) selon l'une quelconque des revendications 8 à 11, dans laquelle la deuxième zone courbée (17) ne comprend pas de trous traversants.

13. Plaque (10) selon l'une quelconque des revendications 8 à 12, dans laquelle l'emplacement (20) du second changement de direction de la seconde partie courbée (17) est à une distance (A2) comprise entre 50 mm et 70 mm, de préférence de 61 mm, de l'extrémité distale (21) de la plaque (10). et/ou
la plaque (10) est configurée de telle sorte que l'emplacement (20) du deuxième changement de direction de la deuxième partie courbée (17) peut être placé à une distance (D2) comprise entre 10 mm et 12 mm, de préférence de 11 mm, de l'extrémité proximale (25) du deuxième os tubulaire (2).

14. Plaque (10) selon l'une quelconque des revendications précédentes, dans laquelle il est prévu dans la zone de fixation proximale (14) et/ou dans la première partie courbée (16) et/ou dans la zone de fixation distale (15) au moins un trou de vis (22) et/ou au moins un trou oblong (23) et/ou au moins un trou de fil K (24).

15. Plaque (10) selon l'une des revendications précédentes, dans laquelle un axe longitudinal de la partie proximale (11) forme un angle de +45° à -45°, de préférence 0°, avec un axe longitudinal de la partie distale (12) en vue de dessus et/ou forme un angle (α) de -45° à +45°, de préférence 12°, en vue latérale.

16. Plaque (10) selon l'une quelconque des revendications précédentes, dans laquelle l'épaisseur de la plaque (10) diminue dans la partie proximale (11) vers l'extrémité proximale (19) et/ou l'épaisseur de la plaque (10) diminue dans la partie distale (12) vers l'extrémité distale (21).
